(19) Europäisches Patentamt
European Patent Office
Office européen des brevets

(11)  **EP 2 510 348 B1**

(12)  **EUROPEAN PATENT SPECIFICATION**

(45) Date of publication and mention
of the grant of the patent:
**12.06.2019 Bulletin 2019/24**

(51) Int Cl.:
**B82Y 15/00** (2011.01)     **G01N 27/30** (2006.01)
**G01N 33/00** (2006.01)

(21) Application number: **10771429.7**

(86) International application number:
**PCT/EP2010/066169**

(22) Date of filing: **26.10.2010**

(87) International publication number:
**WO 2011/069743 (16.06.2011 Gazette 2011/24)**

(54) **ELECTROCHEMICAL SENSING METHOD**

ELEKTROCHEMISCHES MESSVERFAHREN

PROCÉDÉ DE DÉTECTION ÉLECTROCHIMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2009 EP 09178381**

(43) Date of publication of application:
**17.10.2012 Bulletin 2012/42**

(73) Proprietors:
• **Nanocyl S.A.**
**5060 Sambreville (BE)**
• **Université de Bretagne Sud**
**56100 Lorient Cedex (FR)**

(72) Inventors:
• **LUIZI, Frederic**
**B-5020 Malonne (BE)**
• **MEZZO, Luca**
**IT-10076 Nole (TO) (IT)**
• **FELLER, Jean-Fançois**
**F-56530 Queven (FR)**
• **CASTRO, Mickaël**
**F-56100 Lorient (FR)**

(74) Representative: **Nony**
**11 rue Saint-Georges**
**75009 Paris (FR)**

(56) References cited:
**EP-A1- 1 803 763        WO-A2-2008/109968**
**US-A1- 2005 000 830    US-A1- 2009 101 501**
**US-B1- 6 315 956**

• LU J ET AL: "Thermo- and chemo-electrical behaviour of carbon nanotube filled co-continuous conductive polymer nanocomposites (CPC) to develop amperometric sensors", MATERIALS RESEARCH SOCIETY SYMPOSIUM PROCEEDINGS, US, vol. 1143, 1 December 2008 (2008-12-01), pages 53-58, XP009133196, ISSN: 0272-9172
• DATABASE COMPENDEX [Online] ENGINEERING INFORMATION, INC., NEW YORK, NY, US; 2008, LU J ET AL: "Thermo- and chemo-electrical behaviour of carbon nanotube filled co-continuous conductive polymer nanocomposites (CPC) to develop amperometric sensors", Database accession no. E20101512834513

**Description**

**Field of the invention**

The present invention is related to a method for detecting at least one chemical analyte vapour in a gaseous environment.

**State of the art**

Document US 5,417,100 discloses the use of conductive polymers in composites to detect the presence of solvents in the environment. The detection of the solvents is achieved by the swelling of the polymer in contact with said solvents, increasing the space between the polymeric chains. This modification of the configuration of the polymeric chains increases the resistivity of the composite. Multiple conductive polymers can be used together in one system to sense multiple solvents as disclosed by US 5,698,089.

US 5, 672, 297 discloses the use of a conductive filler dispersed in an isolating solvent along with swellable polymer particles. The swelling of the polymer particles induced by the environment reduces the volume of the residual free solvent and the local concentration of the filler, which reaches the percolation threshold. The swelling of the polymeric particles thus induces major changes in the electrical conductivity of the mixture.

Document US 7,342,479 discloses the use of coatings comprising carbon nanotubes as chemical sensors, using changes in electrical resistivity of the coating to determine the concentration of a particular chemical analyte. The coating described in this document uses the intrinsic resistivity variation of the carbon nanotubes embedded in a binder such as a polymer. Such chemical sensors need to be supported and are proposed in the form of coatings on dielectric substrates.

Document US 6,315,956 discloses a sensor for detecting the presence of a chemical analyte. The disclosed sensor comprises a polymer blend comprising a first and a second continuous polymer phase (co-continuous phases). The first continuous polymer phase comprises a conductive filler at a concentration above the percolation threshold, so that said first phase is electrically conductive. The second phase is insulating and selected so that the analyte to be detected is soluble in said second phase. In the presence of the analyte, the absorption of said analyte by the second phase induces the swelling of this phase, inducing a conductivity change of the macroscopic blend. Furthermore, the first polymer phase is insensitive to the analyte.

Lu J. et al., Thermo- and chemo-electrical behaviour of carbon nanotube filled co-continuous conductive polymer nanocomposites (CPC) to develop amperometric sensors, 2008 MRS Fall Meeting, abstract, discloses use of conductive polymer nanocomposite (PA12/PCL-MWNT) of multiwall carbon nanotubes (MWNT) filled with polycaprolactone (PCL) blended with polyamidel2 (PA) for detecting (toluene) vapours via an electrochemical method.

**Aims of the invention**

The present invention aims to provide an electrochemical method for the detection of at least one chemical analyte that overcomes the drawbacks of the prior art.

**Summary of the invention**

The present invention is also related to a method for detecting at least one chemical analyte vapour in a gaseous environment comprising the steps of:

- providing a self-supported fibre-based electrochemical sensor, said fibre-based sensor comprising at least one type of composite fibres, said type of composite fibres comprising a co-continuous phase blend comprising a first and a second continuous polymer phase, the first polymer phase being sensitive to the chemical analyte vapour to be detected in use, wherein said first polymer phase comprises a dispersion of carbon nanotubes at a concentration above the percolation threshold and wherein the chemical analyte is soluble in said first polymer phase;

  - measuring the initial electrical conductivity of the fibre-based sensor;

- bringing said fibre-based sensor into contact with at least one chemical analyte to induce a modification of the electrical conductivity of the fibres;
- measuring the modification of the resulting electrical conductivity of said fibre-based sensor and correlating said modification with the identification of the chemical analyte to be detected,

wherein the viscosity ratio between the first and second polymer phase is comprised between 0.67 and 1.5.

According to particular preferred embodiments, the method of the invention further discloses at least one or a suitable combination of the following features:

- the Flory-Huggins interaction parameter between the chemical analyte and the first polymer phase is smaller than 5, 64 [$J^{1/2}$ cm$^{-3/2}$] ;
- said carbon nanotubes are multiwall carbon nanotubes with a specific surface area between 100 and 400 m$^2$/g;
- said first polymer phase is selected from the group consisting of polycaprolactone, polylactic acid, polyethylene oxide and polymethyl metacrylate;
- said at least one chemical analyte is selected from the group consisting of aromatic solvents, preferably styrene and toluene;
- said at least one chemical analyte is selected from the group consisting of alcohols, preferably methanol;
- said at least one chemical analyte is selected from the group consisting of chlorinated solvents, preferably trichloromethane;
- said second polymer phase is a polyolefin, preferably polyethylene or polypropylene;
- said second polymer phase is polyamide;
- the first polymer phase is polycaprolactone, for the detection of tetrahydrofurane;
- the first polymer phase is polylactic acid for the detection of Styrene;
- the first polymer phase is polyethylene oxide for the detection of methanol;
- the first polymer phase is polymethyl metacrylate for the detection of trichloromethane;
- several different fibre-based electrochemical sensors are provided;
- the viscosity ratio between the first and second polymer phases is comprised between 0,8 and 1,2;
- the co-continuous phase blend further comprises a compatibiliser, preferably, said compatibiliser being selected from the group consisting of maleic anhydride grafted polyolefin, ionnomers and copolymers.

## Brief description of the drawings

Figure 1 represents the spinning process for the production of the fibres used in the fibre-based electrochemical sensor used in the method.

Figure 2 represents a SEM analysis of a transverse section of a fibre comprising a PP / PCL blend at 50/50 wt with 3% CNT (sample 4) dispersed in the PCL phase, after extraction of the PCL phase.

Figure 3 represents a graph of the continuity ratio of PCL measured by selective extraction of PCL using acetic acid.

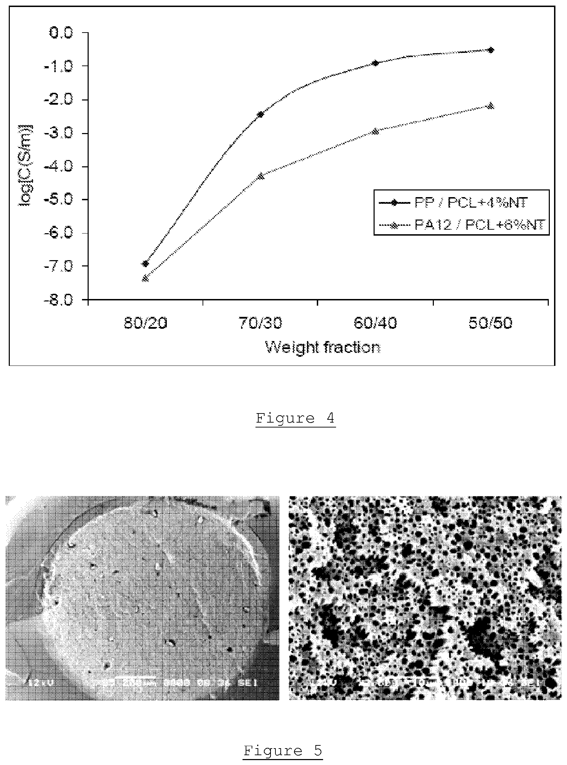

Figure 4 represents the electrical conductivity as a function of the weight fraction of PCL in both PA12 and PP.

Figure 5 represents SEM pictures of PA12/PCL blends at 50/50 wt(sample 9) after extraction of the PCL phase.

Figure 6 represents the change in electrical conductivity (relative amplitude) of fibres of various compositions in the presence of trichloromethane.

Figure 7 represents the change in electrical conductivity (relative amplitude) of fibres of various compositions in the presence of toluene.

Figure 8 represents the change in electrical conductivity (relative amplitude) of fibres of various compositions in the presence of styrene.

Figure 9 represents the change in electrical conductivity (relative amplitude) of fibres of various compositions in the presence of methanol.

Figure 10 represents the change in electrical conductivity of PA12/PMMA fibres (sample 9) in the presence of various vapours of chemical analytes in dynamic mode at 500 cm$^3$.min$^{-1}$ of vapour.

Figure 11 represents the change in electrical conductivity of PP/PCL fibres in the presence of various liquid chemical analytes.

Figure 12 represents the change in electrical conductivity of PA12/PMMA fibres (sample 9) in the presence of various amounts of vapours of chloroform in dynamic mode for various flow rates.

## Detailed description of the invention

The fibre-based sensor suitable for the detection of at least one chemical analyte comprises a blend of at least two co-continuous polymer phases. By co-continuous phase blend, it is meant a phase blend comprising two continuous phases.

The first polymer phase comprises a conductive filler, such as carbon nanotubes (CNT). The chemical analyte to be detected is soluble in the first polymer phase.

By soluble, it is meant in the present invention that at least some of the chemical analytes can be absorbed or expelled by the first polymer phase in response to changes in environmental analyte concentration.

The absorption of the chemical analyte can induce a swelling of said first polymer phase. The induced swelling modifies the contact between the CNTs and therefore produces a measurable change in the electrical conductivity of the fibre.

Alternatively the diffusion of an analyte can be sufficient to induce an interaction of the chains (by Van der Waals forces or Hydrogen bonds) between the chemical analyte and said first polymer phase so that the polymer chains are able to rearrange their configuration. This will result in a movement of CNTs which will disturb the electron conduction. Thus said first polymer phase can be sensitive to poor solvents and give an electric signal even without any observable swelling of the polymer matrix.

The solubility of a solvent in a polymer can for example be deduced from the Flory-Huggins interaction parameter defined by the equation:

$$\kappa_{12} = \frac{V_{seg}\left(\delta_{Tpol} - \delta_{Tsol}\right)^2}{RT}$$

where $\delta_T$ is the total energy from bonds between molecules derived from:

$$\delta_T^2 = \delta_d^2 + \delta_p^2 + \delta_h^2$$

where $\delta_d$ is the energy from dispersion bonds between molecules, $\delta_p$ is the energy from polar bonds between molecules and $\delta_H$ is the energy from hydrogen bonds between molecules. Examples of values of those parameters are given in table 1.

The concentration of the conductive filler in the first polymer phase is, in the absence of the analyte, above the percolation threshold, so that said first polymer phase is conductive. The percolation threshold is the minimum filler concentration at which a continuous electrically conducting path is formed in the composite. This threshold is characterised by a sharp increase of the conductivity of the blend with an increasing filler concentration. Usually, in conductive polymer composites, this threshold is considered to be the concentration of the filler which induces a resistivity below $10^6$ ohm.cm.

**Table 1. Solubility parameters of solvents and polymer and Flory-Huggins interaction parameters**

| | $\Delta_T$ [1] [$J^{1/2}$ cm$^{-3/2}$] | $\delta_d$ [2] [$J^{1/2}$ cm$^{-3/2}$] | $\delta_p$ [3] [$J^{1/2}$ cm$^{-3/2}$] | $\delta_h$ [4] [$J^{1/2}$ cm$^{-3/2}$] | Mol. vol. [cm$^3$ mol$^{-1}$] | $\chi_{12}$ [5] (PCL/ solvent) | $\chi_{12}$ (PC/ solvent) | $\chi_{12}$ (PMMA/ solvent) | $\chi_{12}$ PEO/ solvent) | $\chi_{12}$ (PLA/ solvent) |
|---|---|---|---|---|---|---|---|---|---|---|
| Water | 47.9 | 15.5 | 16 | 42.4 | 18.1 | 5.09 | 5.64 | 5.6 | 4.82 | 4.38 |
| Methanol | 29.7 | 15.1 | 12.3 | 22.3 | 40.5 | 1.10 | 1.51 | 1.48 | 0.92 | 0.65 |
| Toluene | 18.2 | 18 | 1.4 | 2 | 106.3 | 0.47 | 0.15 | 0.17 | 0.69 | 1.16 |
| Tetrahydrofuran (THF) | 19.4 | 16.8 | 5.7 | 8 | 81.4 | 0.14 | 0.02 | 0.02 | 0.26 | 0.53 |
| Chloroform | 19 | 17.8 | 3.1 | 5.7 | 79.7 | 0.2 | 0.04 | 0.05 | 0.33 | 0.62 |
| Dichloromethan | 9.9 | 8.9 | 3.1 | 3.0 | 65.3 | 3.5 | 5.64 | 2.79 | 3.99 | 4.80 |
| Styrene | 19 | 18.6 | 1 | 4.1 | 114.5 | 0.288 | 0.06 | 0.07 | 0.47 | 0.89 |
| Poly (oxyethylene) | 22.2 | 17.2 | 10.8 | 9 | 37 | - | - | - | - | - |
| Poly(lactic acid) | 23.4 | 15.4 | 15 | 9.3 | 57 | - | - | - | - | - |
| Poly(methyl methacrylate) | 20.2 | 17.02 | 5.8 | 9.2 | 84 | - | - | - | - | - |
| Poly(carbonate) | 20.1 | 18.7 | 2.9 | 7 | 203 | - | - | - | - | - |
| Poly (caprolactone) | 21.5 | 18.1 | 9 | 7.2 | 96 | - | - | - | - | - |

[1] $\delta_T$: The total energy from bonds between molecules derived from $\delta_T^2 = \delta_d^2 + \delta_p^2 + \delta_h^2$ .

[2] $\delta_d$: The energy from dispersion bonds between molecules

[3] $\delta_p$: The energy from polar bonds between molecules

[4] $\delta_H$: The energy from hydrogen bonds between molecules

[5] $\chi_{12}$: Flory-Huggins interaction parameter defined by the equation $\kappa_{12} = \dfrac{V_{seg}\left(\delta_{Tpol} - \delta_{Tsol}\right)^2}{RT}$

The first polymer phase is selected in such a way that it has a good compatibility with the analyte to be detected (i.e. the analyte can be absorbed). For example, in order to detect the vapour of a solvent, the first polymer phase will be selected so that the Flory Huggins interaction parameter between the analyte to be detected and the first polymer phase is smaller than about 6[$J^{1/2}$ cm$^{-3/2}$], preferably smaller than 5,64 [$J^{1/2}$ cm$^{-3/2}$].

Due to the high compatibility of the first polymer phase with the chemical analyte, large amounts of this chemical analyte can diffuse through and be absorbed by said first polymer phase, resulting in a modification of the local mobility of the polymer chain, inducing a CNT reorganisation resulting in a modification of the macroscopic resistivity of the fibre. This diffusion may entail a swelling of the first polymer phase.

At high analyte concentrations, the swelling can be so high that the mechanical properties of the first phase severely drop. For that reason, a supporting material is necessary to maintain the mechanical integrity of the fibre. This supporting material is formed by the second polymer phase. Said second polymer phase is selected to be almost insensitive to the chemical analyte and is nonconductive. The second polymer phase also act as a protective envelope which, in use, preserves the fibre integrity.

Preferably, when used in a liquid environment, the second polymer phase is selected from the group consisting of polyolefin, more preferably polypropylene or polyethylene.

Preferably, when used in a gaseous environment, the second polymer phase is based upon polyamide.

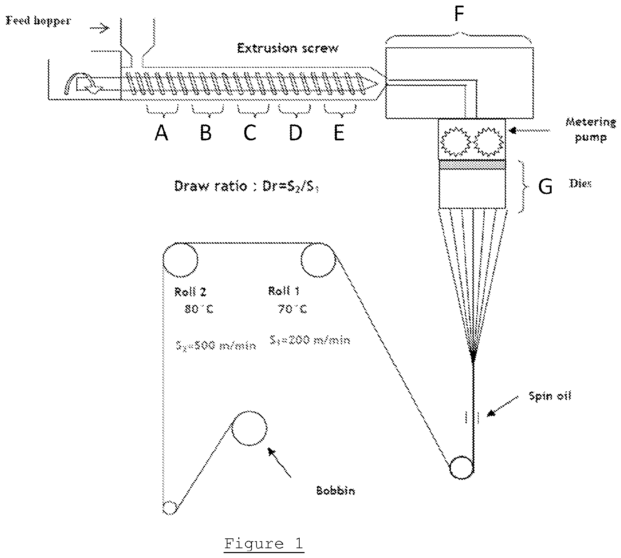

The fibres are produced in a spinning system, as shown in fig. 1. The use of fibres brings several advantages: the surface to volume ratio can be optimized by using several fibres in bundles, thus reducing the response time delay in resistivity measurements, the fibres can be included in smart textile, they can easily be shaped in particular geometrical forms, etc.

The compatibility of the polymer blend has an impact on the spinnability of the biphasic systems. More particularly, the adhesion between both phases improves the spinnability of the blend. This adhesion can be achieved either by the selection of intrinsically adhering pairs of polymers, or by addition of a compatibilizer in one of the polymer phases. Examples of compatibilizers are maleic anhydride grafted polyolefins, ionomers, bloc copolymers comprising a bloc of each phase, etc. This cohesion has also an impact on the blend morphology.

To enable the co-continuity of phases, the ratio of viscosities between the two phases of the biphasic system is comprised between 0.67 and 1.5, more preferably comprised between 0.8 and 1.2, ideally, close to 1. The other parameters determining the co-continuity are the nature of the polymers (viscosities, interfacial tension and the ratio of these viscosities), their volume fractions and the processing conditions.

A convenient way to evaluate viscosities in the present description is the so called MFI procedure described in the ISO 1133:1997, at a temperature close to the extrusion temperature of the fibres (i.e. 190°C for PE and 230°C for PP as an example). Such MFI measurements are usually good relative estimators of the real viscosity in the usual extrusion conditions. Alternatively, the viscosities may be measured dynamically according to ISO 11443 under shear conditions close to the extrusion conditions. But such methods are much more difficult to use, and, as already stated, as far as only viscosity ratio are needed, MFI represent sufficient estimators.

**Examples**

The examples presented are related to blends comprising:

- Poly(ε-caprolactone) (PCL), polyethylene oxide (PEO), polylactic acid (PLA) and polymethyl metacrylate (PMMA) as the first polymer phase;
- polypropylene (PP) and polyamide 12 (PA12) as the second polymer phase;
- Carbon Nanotubes (CNT)

PCL, namely CAPA 6800 from Solvay, is a biodegradable polymer with a relatively low melting temperature of about 60°C. The polyethylene oxide was provided by Sima Aldrich, the grade name was PEO 181986. The PMMA used was VQ101S provided by Rhöm. PLA was the grade L-9000 commercialised by Biomer.

PP of the type H777-25R from DOW was chosen. PA12 was Grilamid L16E from EMS-Chemie. These PP and PA12 are spinning types and should lead to a good spinnability of the blends.

Composites of these polymers with various weight contents of carbon nanotubes (CNT) from Nanocyl were prepared with various weight fractions. Carbon nanotubes are multiwall carbon nanotubes with a diameter between 5 and 20 nm, preferably between 6 and 15 nm, and a specific surface area between 100 m$^2$/g and 400 m$^2$/g.

The production of the fibres was done in a two step process. In a first step, the carbon nanotubes were dispersed in the first polymer in a twin-screw compounding extruder. The obtained extrudates are then pelletized and dry blended with the second polymer.

The obtained dry blend was then fed in the hopper of a single screw extruder, feeding a spinning die as represented in fig. 1. The temperatures in the various zones corresponding to fig. 1 are summarised in table 2. The temperatures were fixed for a given second polymer phase.

Table 2: Temperatures in C in the different extrusion zones corresponding to fig.1

| First polymer | A | B | C | D | E | F | G |
|---|---|---|---|---|---|---|---|
| PP | 180 | 190 | 200 | 210 | 230 | 230 | 230 |
| PA12 | 180 | 185 | 190 | 195 | 200 | 200 | 200 |

The composition of the conductive polymer composites (CPC) prepared for further experiments are detailed in Table 3.

Table 3: CPC compositions used in co-continuity and conductivity experiments.

| | polymer blend | polymer phase weight fraction : first polymer/ second polymer | CNT weight fractions in the first polymer phase in wt % |
|---|---|---|---|
| Sample 1 | PCL/PP | 80/20 | 3 |
| Sample 2 | PCL/PP | 70/30 | 3 |
| Sample 3 | PCL/PP | 60/40 | 3 |
| Sample 4 | PCL/PP | 50/50 | 3 |
| Sample 5 | PCL/PA12 | 80/20 | 6 |
| Sample 6 | PCL/PA12 | 70/30 | 6 |
| Sample 7 | PCL/PA12 | 60/40 | 6 |
| Sample 8 | PCL/PA12 | 50/50 | 6 |
| Sample 9 | PMMA/PA12 | 50/50 | 2 |
| Sample 10 | PLA/PA12 | 50/50 | 2 |
| Sample 11 | PEO/PA12 | 50/50 | 3 |
| Sample 12 | PCL/PA12 | 50/50 | 3 |

A melt spinning machine (Spinboy I manufactured by Busschaert Engineering) was used to obtain the multifilament yarns. The multifilament yarns are covered with a spin finish, rolled up on two heated rolls with varying speeds (S1 and S2) to regulate the drawing ratio. The theoretical drawing of multifilament is given by the ratio DR = S2/S1. During the fibre spinning, the molten polymer containing nanotubes is forced through a die head with a rather low diameter (400 $\mu$m or 1.2 mm depending on the polymer) and through a series of filters. Several parameters were optimized during the process to obtain spinnable blends. These parameters were mainly the temperature of the heating zones, the speed of the volume metering pump and the roll speed.

**Determination of PCL phase continuity by selective extraction**

An extended study of the co-continuity of the PP/PCL and PA12/PCL blends has been performed. The selective extraction of one phase provides a good estimation of the co-continuity of a mixture. This is achieved by the dissolution of PCL into acetic acid, this solvent having no effect on PA12 and PP. If the mixture has a nodular structure, PCL inclusions will not be affected by the solvent, so they are not dissolved. The percentage of the PCL phase continuity is then deduced by weight loss measurement.

To remove PCL, fibres of each blend were immersed in acetic acid for 2 days at room temperature. The extracted strands were then rinsed in acetic acid and dried at 50°C to remove the acetic acid. After repeating the extraction process several times, the specimen weight converges toward a constant value.

The phase continuity was calculated using the ratio of the soluble PCL part to the initial PCL concentration in the blend, where the dissolvable PCL part is the weight difference of the sample before and after extraction.

The PCL part in the blend is calculated using the following equation:

```
% Continuity of PCL = ((Weight PCLinitial - Weight
PCLfinal) / Weight PCLinitial)* 100%
```

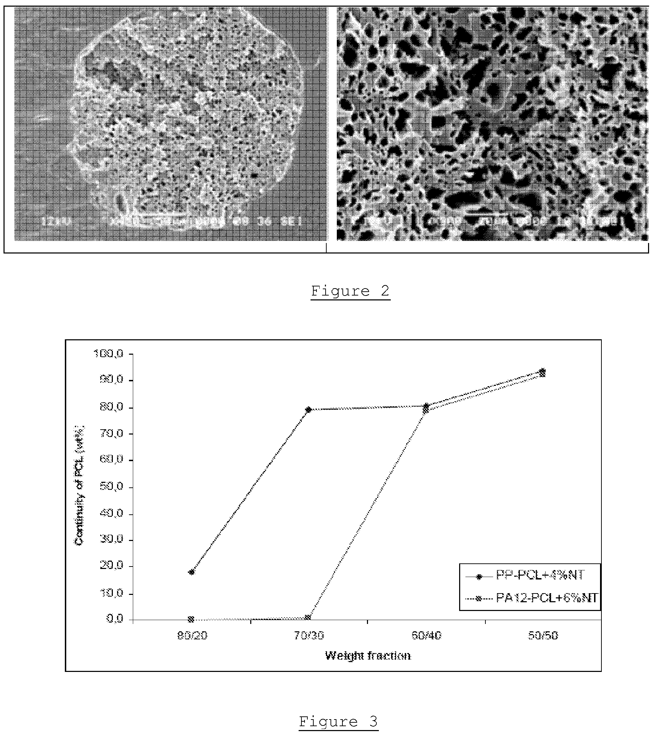

The results are represented in fig. 3. It can be seen in that figure that the continuity of PCL is reached around 40% PCL in PA12 and 30% PCL in PP.

**Vapour sensing**

In the so-called static mode of analysis, the desired amount of solvent molecules to analyse is introduced in a closed chamber. In saturated conditions the solvent is present in a liquid state in the bottom of the chamber. The vapour sensing behaviours of several samples were investigated for toluene, styrene, trichloromethane, THF, water and methanol vapour. Electrical resistance measurements were performed with a Keithley multimeter 2000 at room temperature. Each sample was exposed to saturated organic vapours for 300 s and then moved into a dessicator with dried air atmosphere. Those cycles were repeated three times. The resistance of the fibre was measured automatically every 10s. The relative amplitude was then defined as (R- R0)/ R0, where R0 is the initial resistance of the composite (i.e. when exposed to air atmosphere).

In the so-called dynamic mode, electrochemical properties of the composite fibres comprising CNTs were investigated by recording their electrical responses when submitted to 15 min of successive cycles of nitrogen and vapour streams. The dynamic system consisting in mass flow controllers, solvent bubblers and electrical valves is controlled by the LabView software. Bubbling $N_2$ gas in liquid solvent provided a saturated vapour stream, which was in turn diluted by a second $N_2$ flow to the desired concentration at room temperature. The design of the device allows to keep constant the total flow rate $Q_v$ ($cm^3.min^{-1}$), while the analyte flow rate is adapted to investigate the effect of the analyte content. Electrical characteristics of the CPC transducer were recorded with a KEITHLEY 6517A multimeter. Samples were placed in a chamber of 9 cm $\times$ 3 cm $\times$ 3.5 cm.

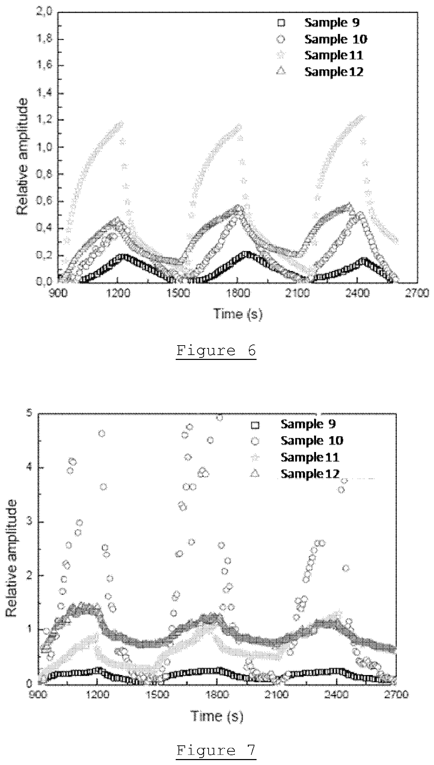

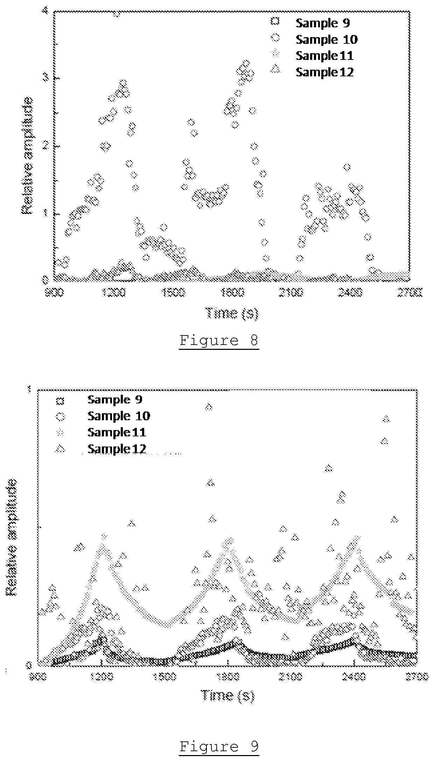

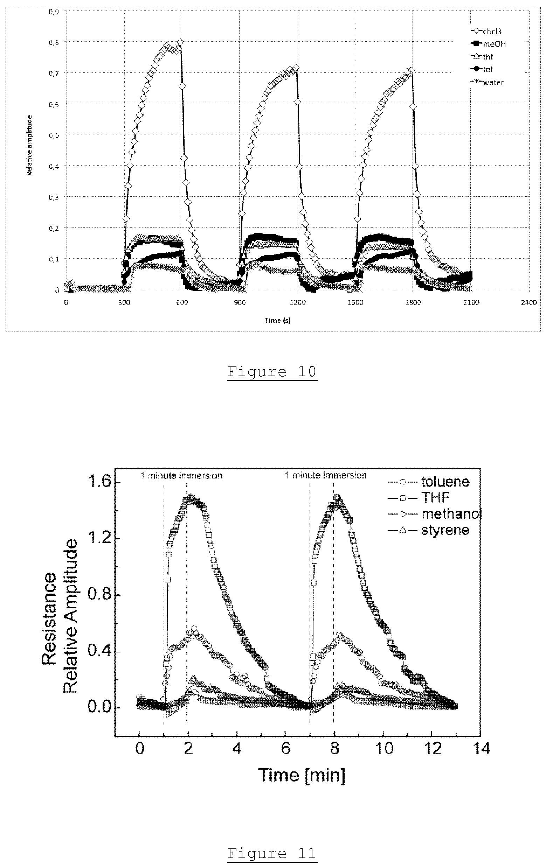

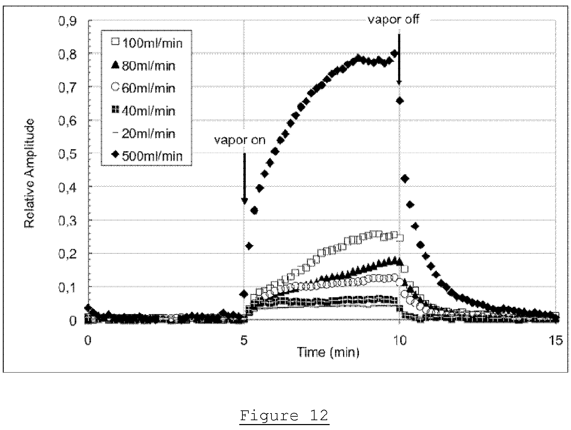

The relative amplitudes obtained with the different samples and different solvents are represented in fig. 6 to 12. It can be seen in those figures that each particular first polymer phase has a specific sensitivity towards a particular solvent. For example, PLA shows a better sensitivity to styrene vapour and PEO shows a better sensitivity to methanol vapour. Those differences can advantageously be used in vapour sensing devices comprising several sensing fibres for improving the selectivity of the devices towards particular chemicals.

**Claims**

1. Method for detecting at least one chemical analyte vapour in a gaseous environment comprising the steps of:

   - providing a self-supported fibre-based electrochemical sensor, said fibre-based sensor comprising at least one type of composite fibres, said type of composite fibres comprising a co-continuous phase blend comprising a first and a second continuous polymer phase, the first polymer phase being sensitive to the chemical analyte vapour to be detected in use, wherein said first polymer phase comprises a dispersion of carbon nanotubes at a concentration above the percolation threshold and wherein the chemical analyte is soluble in said first polymer phase;
   - measuring the initial electrical conductivity of the fibre-based sensor;
   - bringing said fibre-based sensor into contact with at least one chemical analyte to induce a modification of the electrical conductivity of the fibres;
   - measuring the modification of the resulting electrical conductivity of said fiber based sensor and correlating said modification with the identification of the chemical analyte to be detected,

   wherein the viscosity ratio between the first and second polymer phases is comprised between 0.67 and 1.5.

2. Method according to claim 1, wherein the Flory-Huggins interaction parameter between the chemical analyte and the first polymer phase is smaller than 5,64 [J1/2cm-3/2].

3. Method according to claim 1 or 2, wherein said carbon nanotubes are multiwall carbon nanotubes with a specific surface area between 100 and 400 m $^2$ /g.

4. Method according to claim 1, 2 or 3, wherein said first polymer phase is selected from the group consisting of polycaprolactone, polylactic acid, polyethylene oxide and polymethyl metacrylate.

5. Method according to any of the previous claims, wherein said at least one chemical analyte is selected from the group consisting of aromatic solvents, preferably styrene and toluene.

6. Method according to any of the previous claims, wherein said at least one chemical analyte is selected from the group consisting of alcohols, preferably methanol.

7. Method according to any of the previous claims, wherein said at least one chemical analyte is selected from the group consisting of chlorinated solvents, preferably trichloromethane.

8. Method according to any of the previous claims, wherein said second polymer phase is a polyolefin, preferably polyethylene or polypropylene.

9. Method according to any of the previous claims, wherein said second polymer phase is polyamide.

10. Method according to any of the previous claims, wherein the first polymer phase is polycaprolactone, for the detection of tetrahydrofurane.

11. Method according to any of the previous claims, wherein the first polymer phase is polylactic acid for the detection of Styrene.

12. Method according to any of the previous claims, wherein the first polymer phase is polyethylene oxide for the detection of methanol.

13. Method according to any of the previous claims, wherein the first polymer phase is polymethylmetacrylate for the detection of trichloromethane.

14. Method according to any of the previous claims, wherein the viscosity ratio between the first and second polymer phases is comprised between 0,8 and 1,2.

15. Method according to any of the previous claims, wherein the co-continuous phase blend further comprises a compatibiliser selected from the group consisting of maleic anhydride grafted polyolefin, ionnomers and bloc copolymers comprising a bloc of each phase.


**Patentansprüche**

1. Verfahren zum Detektieren von mindestens einem chemischen Analytdampf in einer gasförmigen Umgebung, umfassend die Schritte:

   - Bereitstellen eines selbsttragenden faserbasierten elektrochemischen Sensors, wobei der faserbasierte Sensor mindestens einen Typ von Verbundfasern umfasst, wobei der Typ von Verbundfasern ein cokontinuierliches Phasengemisch umfasst, das eine erste und eine zweite kontinuierliche Polymerphase umfasst, wobei die erste Polymerphase gegenüber dem bei Gebrauch zu detektierenden chemischen Analytdampf sensitiv ist, wobei die erste Polymerphase eine Dispersion von Kohlenstoffnanoröhrchen in einer Konzentration oberhalb des Perkolationsschwellenwerts umfasst, und wobei der chemische Analyt in der ersten Polymerphase löslich ist;
   - Messen der anfänglichen elektrischen Leitfähigkeit des faserbasierten Sensors;
   - Bringen des faserbasierten Sensors in Kontakt mit mindestens einem chemischen Analyten, um eine Modifizierung der elektrischen Leitfähigkeit der Fasern zu induzieren;
   - Messen der Modifizierung der resultierenden elektrischen Leitfähigkeit des faserbasierten Sensors und Korrelieren der Modifizierung mit der Identifizierung des zu detektierenden chemischen Analyten,

   wobei das Viskositätsverhältnis zwischen der ersten und der zweiten Polymerphase zwischen 0,67 und 1,5 liegt.

2. Verfahren nach Anspruch 1, wobei der Flory-Huggins-Wechselwirkungsparameter zwischen dem chemischen Analyten und der ersten Polymerphase kleiner als 5,64 [J1/2cm-3/2] ist.

3. Verfahren nach Anspruch 1 oder 2, wobei die Kohlenstoffnanoröhrchen mehrwandige Kohlenstoffnanoröhrchen mit einer spezifischen Oberfläche zwischen 100 und 400 m$^2$/g sind.

4. Verfahren nach Anspruch 1, 2 oder 3, wobei die erste Polymerphase ausgewählt ist aus der Gruppe bestehend aus Polycaprolacton, Polymilchsäure, Polyethylenoxid und Polymethylmethacrylat.

5. Verfahren nach einem der vorhergehenden Ansprüche, wobei der mindestens eine chemische Analyt ausgewählt ist aus der Gruppe bestehend aus aromatischen Lösungsmitteln, vorzugsweise Styrol und Toluol.

6. Verfahren nach einem der vorhergehenden Ansprüche, wobei der mindestens eine chemische Analyt ausgewählt ist aus der Gruppe bestehend aus Alkoholen, vorzugsweise Methanol.

7. Verfahren nach einem der vorhergehenden Ansprüche, wobei der mindestens eine chemische Analyt ausgewählt ist aus der Gruppe bestehend aus chlorierten Lösungsmitteln, vorzugsweise Trichlormethan.

8. Verfahren nach einem der vorhergehenden Ansprüche, wobei die zweite Polymerphase ein Polyolefin ist, vorzugsweise Polyethylen oder Polypropylen.

9. Verfahren nach einem der vorhergehenden Ansprüche, wobei die zweite Polymerphase Polyamid ist.

10. Verfahren nach einem der vorhergehenden Ansprüche, wobei die erste Polymerphase Polycaprolacton zur Detektierung von Tetrahydrofuran ist.

11. Verfahren nach einem der vorhergehenden Ansprüche, wobei die erste Polymerphase Polymilchsäure zur Detektierung von Styrol ist.

12. Verfahren nach einem der vorhergehenden Ansprüche, wobei die erste Polymerphase Polyethylenoxid zur Detektierung von Methanol ist.

13. Verfahren nach einem der vorhergehenden Ansprüche, wobei die erste Polymerphase Polymethylmethacrylat zur Detektierung von Trichlormethan ist.

14. Verfahren nach einem der vorhergehenden Ansprüche, wobei das Viskositätsverhältnis zwischen der ersten und der zweiten Polymerphase zwischen 0,8 und 1,2 liegt.

15. Verfahren nach einem der vorhergehenden Ansprüche, wobei das cokontinuierliche Phasengemisch ferner einen Verträglichmacher umfasst, der ausgewählt ist aus der Gruppe bestehend aus mit Maleinsäureanhydrid gepfropftem Polyolefin, Ionomeren und Blockcopolymeren, die einen Block von jeder Phase umfassen.

**Revendications**

1. Procédé pour la détection d'au moins une vapeur d'analyte chimique dans un environnement gazeux comprenant les étapes consistant à :

   - disposer d'un détecteur électrochimique à base de fibres autoportant, ledit détecteur à base de fibres comprenant au moins un type de fibres composites, ledit type de fibres composites comprenant un mélange de phases co-continues comprenant des première et seconde phases continues de polymère, la première phase de polymère étant sensible à la vapeur d'analyte chimique à détecter lors de l'utilisation, ladite première phase de polymère comprenant une dispersion de nanotubes de carbone à une concentration au-dessus du seuil de percolation et l'analyte chimique étant soluble dans ladite première phase de polymère ;
   - mesurer la conductivité électrique initiale du détecteur à base de fibres ;
   - mettre en contact ledit détecteur à base de fibres avec au moins un analyte chimique pour provoquer une modification de la conductivité électrique des fibres ;
   - mesurer la modification de la conductivité électrique résultante dudit détecteur à base de fibres et corréler ladite modification avec l'identification de l'analyte chimique à détecter,

   dans lequel le rapport des viscosités entre les première et seconde phases de polymère est compris entre 0,67 et 1,5.

2. Procédé selon la revendication 1, dans lequel le paramètre d'interaction de Flory-Huggins entre l'analyte chimique et la première phase de polymère est plus petit que 5,64 [J1/2cm-3/2].

3. Procédé selon la revendication 1 ou 2, dans lequel lesdits nanotubes de carbone sont des nanotubes de carbone multifeuillets ayant une surface spécifique entre 100 et 400 m$^2$/g.

4. Procédé selon la revendication 1, 2 ou 3, dans lequel ladite première phase de polymère est choisie dans le groupe constitué par de la polycaprolactone, du poly(acide lactique), du poly(oxyde d'éthylène) et du poly(méthacrylate de méthyle).

5. Procédé selon l'une quelconque des revendications précédentes, dans lequel ledit au moins un analyte chimique est choisi dans le groupe constitué par les solvants aromatiques, de préférence le styrène et le toluène.

6. Procédé selon l'une quelconque des revendications précédentes, dans lequel ledit au moins un analyte chimique est choisi dans le groupe constitué par les alcools, de préférence le méthanol.

7. Procédé selon l'une quelconque des revendications précédentes, dans lequel ledit au moins un analyte chimique est choisi dans le groupe constitué par les solvants chlorés, de préférence le trichlorométhane.

8. Procédé selon l'une quelconque des revendications précédentes, dans lequel ladite seconde phase de polymère est une polyoléfine, de préférence du polyéthylène ou du polypropylène.

9. Procédé selon l'une quelconque des revendications précédentes, dans lequel ladite seconde phase de polymère est du polyamide.

10. Procédé selon l'une quelconque des revendications précédentes, dans lequel la première phase de polymère est de la polycaprolactone, pour la détection de tétrahydrofurane.

11. Procédé selon l'une quelconque des revendications précédentes, dans lequel la première phase de polymère est du poly(acide lactique) pour la détection de styrène.

12. Procédé selon l'une quelconque des revendications précédentes, dans lequel la première phase de polymère est du poly(oxyde d'éthylène) pour la détection de méthanol.

13. Procédé selon l'une quelconque des revendications précédentes, dans lequel la première phase de polymère est du poly(méthacrylate de méthyle) pour la détection de trichlorométhane.

14. Procédé selon l'une quelconque des revendications précédentes, dans lequel le rapport des viscosités entre les première et seconde phases de polymère est compris entre 0,8 et 1,2.

15. Procédé selon l'une quelconque des revendications précédentes, dans lequel le mélange de phases co-continues comprend en outre un agent de compatibilité choisi dans le groupe constitué par une polyoléfine greffée par de l'anhydride maléique, les ionomères et les copolymères séquencés comprenant une séquence de chaque phase.

Figure 1

Figure 2

Figure 3

Figure 4

Figure 5

Figure 6

Figure 7

Figure 8

Figure 9

Figure 10

Figure 11

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5417100 A
- US 5698089 A
- US 5672297 A
- US 7342479 B
- US 6315956 B

### Non-patent literature cited in the description

- **LU J. et al.** Thermo- and chemo-electrical behaviour of carbon nanotube filled co-continuous conductive polymer nanocomposites (CPC) to develop ampero- metric sensors. *MRS Fall Meeting,* 2008